(19) Europäisches Patentamt
European Patent Office
Office européen des brevets

(11) Veröffentlichungsnummer: **0 299 370 B1**

(12) **EUROPÄISCHE PATENTSCHRIFT**

(45) Veröffentlichungstag der Patentschrift: **31.08.94**

(51) Int. Cl.5: **C07C 305/10**, C08G 65/32, C11D 1/29

(21) Anmeldenummer: **88110904.5**

(22) Anmeldetag: **08.07.88**

# (54) Sulfatierte Hydroxy-Mischether, Verfahren zu ihrer Herstellung und ihre Verwendung.

(30) Priorität: **15.07.87 DE 3723354**

(43) Veröffentlichungstag der Anmeldung:
**18.01.89 Patentblatt 89/03**

(45) Bekanntmachung des Hinweises auf die Patenterteilung:
**31.08.94 Patentblatt 94/35**

(84) Benannte Vertragsstaaten:
**DE GR**

(56) Entgegenhaltungen:
**EP-A- 0 167 337**
**DE-A- 1 910 765**
**DE-A- 2 324 894**
**DE-A- 3 345 349**

(73) Patentinhaber: **Henkel Kommanditgesellschaft auf Aktien**

**D-40191 Düsseldorf (DE)**

(72) Erfinder: **Schenker, Gilbert, Dr.**
**Hermann-Hesse-Strasse 5**
**D-4006 Erkrath 2 (DE)**
Erfinder: **Piorr, Robert, Dr.**
**Kieselei 12**
**D-4000 Düsseldorf (DE)**
Erfinder: **Lüttge, Sabine**
**Marktfelderstrasse 30**
**D-4050 Mönchengladbach 1 (DE)**

**EP 0 299 370 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft sulfatierte Hydroxyalkylpolyethylenglykolether und ihre Gemische, Verfahren zur Herstellung derartiger Verbindungen bzw. ihrer Gemische und ihre Verwendung als Netzmittel und Waschmittelrohstoffe.

Hydroxyalkylpolyethylenglykolether der allgemeinen Formel (I)

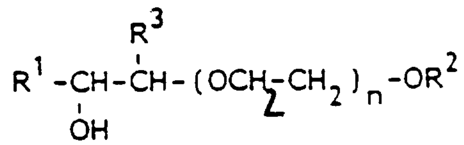

(I)

sind aus dem Stand der Technik bekannt. So werden in der DE-OS 33 45 349 Verbindungen der allgemeinen Formel (I), in der

$R^1$ einen geradkettigen Alkylrest mit 6 bis 16 C-Atomen.

$R^2$ einen geradkettigen oder verzweigten Alkylrest mit 4 bis 8 C-Atomen.

$R^3$ Wasserstoff oder einen Alkylrest mit 1 bis 8 C-Atomen, und

n eine Zahl von 7 bis 12

bedeuten, mit der Maßgabe, daß die Gesamtzahl der in $R^1$ und $R^3$ enthaltenen C-Atome 6 bis 16 beträgt, sowie die Verwendung derartiger Verbindungen als schaumdrückende Zusätze für schaumarme Reinigungsmittel beschrieben.

Des weiteren sind aus der Europäischen Patentanmeldung EP-A2 0 167 337 Alkylethersulfate bekannt, die man durch Sulfatierung von Anlagerungsprodukten von 2 bis 10 Mol eines $C_2$-$C_4$-Alkylenoxids an lineare oder verzweigte Alkohole mit 8 bis 10 Kohlenstoffatomen, und nachfolgende Neutralisation erhält.

Es wurde gefunden, daß man Hydroxyalkylpolyethylenglykolether des Typs (I) mit guten Ergebnissen sulfatieren kann und dabei sulfatierte Hydroxy-Mischether erhält, die sich nicht nur hervorragend als Netzmittel und Waschmittelrohstoffe mit geringer Schaumneigung eignen, sondern auch hinsichtlich ihrer Toxizität völlig unbedenklich sind und den geltenden Vorschriften zu entsprechen vermögen. Die erfindungsgemäßen Verbindungen lassen sich auch weitgehend biologisch abbauen, so daß keine ökologischen Bedenken gegen ihre Verwendung bestehen.

Die Erfindung betrifft sulfatierte Hydroxyalkylpolyethylenglykolether der allgemeinen Formel (II)

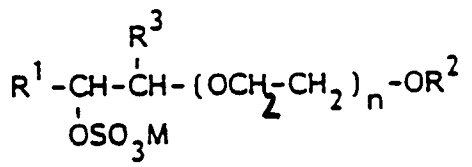

(II)

in der

$R^1$ für Wasserstoff oder einen linearen Alkylrest mit 6 bis 16 C-Atomen,

$R^2$ für einen linearen oder verzweigten, gesättigten Alkylrest mit 1 bis 22 C-Atomen,

$R^3$ für Wasserstoff oder einen linearen Alkylrest mit 1 bis 16 C-Atomen,

M für Wasserstoff, Ammonium, Alkylammonium, Alkanolammonium, worin die Alkyl- und Alkanolreste je 1 bis 4 C-Atome haben, oder ein einwertiges Metallatom und

n für eine Zahl im Bereich von 1 bis 30

stehen, mit der Maßgabe daß die Gesamtzahl der in $R^1$ und $R^3$ enthaltenen C-Atome 6 bis 16 beträgt.

Die Erfindung betrifft außerdem ein Verfahren zur Herstellung von sulfatierten Hydroxyalkylpolyethylenglykolethern der allgemeinen Formel (II)

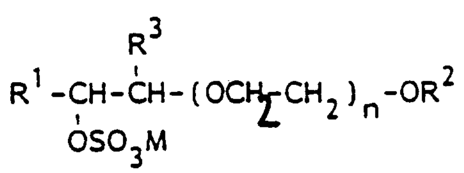

(II)

in der
$R^1$ für Wasserstoff oder einen linearen Alkylrest mit 6 bis 16 C-Atomen,
$R^2$ für einen linearen oder verzweigten, gesättigten Alkylrest mit 1 bis 22 C-Atomen,
$R^3$ für Wasserstoff oder einen linearen Alkylrest mit 1 bis 16 C-Atomen,
M für Wasserstoff, Ammonium, Alkylammonium, Alkanolammonium, worin die Alkyl- und Alkanolreste je 1 bis 4 C-Atome haben, oder ein einwertiges Metallatom und
n für eine Zahl im Bereich von 1 bis 30
stehen, mit der Maßgabe, daß die Gesamtzahl der in $R^1$ und $R^3$ enthaltenen C-Atome 6 bis 16 beträgt bei dem man a) Epoxide der allgemeinen Formel (III)

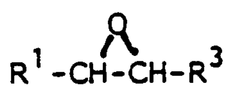

(III)

in der $R^1$ und $R^3$ die oben angegebenen Bedeutungen haben, mit Alkoholethoxylaten der allgemeinen Formel (IV)

$R^2-O(CH_2CH_2O)_nH$ (IV)

in der $R^2$ und n die oben angegebenen Bedeutungen haben, bei erhöhter Temperatur in Gegenwart eines Katalysators umsetzt, b) das Reaktionsprodukt bei erhöhter Temperatur mit einem Sulfatierungsreagenz behandelt, c) das Sulfatierungsprodukt in eine wäßrige basische Lösung einträgt, das Gemisch bei erhöhter Temperatur hält, und auschließend das Gemisch auf einen pH-Wert im neutralen bzw. schwach alkalischen Bereich einstellt.

Die Erfindung betrifft außerdem die Verwendung der sulfatierten Hydroxyalkylpolyethylenglykolether der allgemeinen Formel (II) oder ihrer Mischungen als Netzmittel und als Waschmittelrohstoffe. -

Die erfindungsgemäßen sulfatierten Hydroxyalkylpolyethylenglykolether entsprechen der allgemeinen Formel (II)

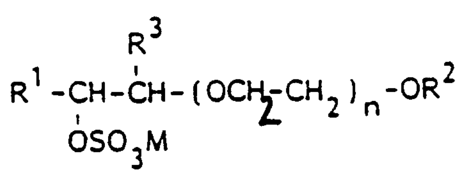

(II)

In der allgemeinen Formel (II) steht $R^1$ für einen linearen Alkylrest mit 6 bis 16 C-Atomen. Es kommen also für die erfindungsgemäßen Verbindungen als Substituent $R^1$ die Reste n-Hexyl, n-Heptyl, n-Octyl, n-Nonyl, n-Decyl, n-Undecyl, n-Dodecyl, n-Tridecyl, n-Tetradecyl, n-Pentadecyl und n-Hexadecyl in Frage.

Die Bedeutung von $R^3$ in der oben genannten allgemeinen Formel (II) ist Wasserstoff oder ein linearer Alkylrest mit 1 bis 16 C-Atomen. Neben Wasserstoff kommen also für $R^3$ dieselben Alkylreste wie für $R^1$ in Frage.

In bevorzugten Hydroxyalkylpolyethylenglykolethern der allgemeinen Formel (II) steht $R^1$ für lineare Alkylreste mit 8 bis 12 C-Atomen und $R^3$ Wasserstoff.

In der oben genannten allgemeinen Formel (II) steht $R^2$ erfindungsgemäß für einen linearen oder verzweigten, gesättigten Alkylrest mit 1 bis 22 C-Atomen. Es kommen somit als Substituent $R^2$ die Reste Methyl, Ethyl, n-Propyl, n-Butyl, n-Pentyl. n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octydecyl, n-Nonadecyl, n-Eicosyl, n-Heneicosyl und n-Docosyl sowie die verzweigtkettigen Isomeren der genannten Alkylreste in Frage.

In einer bevorzugten Ausführungsform steht $R^2$ für lineare, gesättigte Alkylreste mit 1 bis 22 C-Atomen.

Eine besonders bevorzugte Ausführungsform umfaßt sulfatierte Hydroxyalkylpolyethylenglykolether der allgemeinen Formel (II), in der $R^2$ für lineare, gesättigte Alkylreste mit 1 bis 12 C-Atomen, bevorzugt für solche Alkylreste mit 1 bis 4 C-Atomen, steht.

In der allgemeinen Formel (II) hat n die Bedeutung einer Zahl von 1 bis 30, wobei der Bereich von 1 bis 12 für n bevorzugt ist. Dies bedeutet, daß in den erfindungsgemäßen Hydroxyalkylpolyethylenglykolethern 1 bis 30 Ethoxyreste, bevorzugt 1 bis 12 Ethoxyreste in die Molekülkette eingebaut sind.

Die Bedeutung von M in der oben genannten allgemeinen Formel (II) ist Wasserstoff. Ammonium, Alkylammonium, Alkanolammonium oder ein einwertiges Metall, wobei die Alkyl- und Alkanolreste der genannten organischen Ammoniumionen je 1 bis 4 C-Atome haben können. In Verbindungen der allgemeinen Formel (II), die gemäß der Erfindung bevorzugt sind, steht M für ein Alkalimetall, wobei besonders bevorzugt solche Verbindungen (II) sind, in denen M für Natrium oder Kalium steht.

Ein Vorteil der erfindungsgemäßen Verbindungen gegenüber anderen eingesetzten Netzmitteln und Waschmittelrohstoffen aus dem Stand der Technik ist, daß sie nicht zur Schaumbildung neigen und ein vergleichbares, wenn nicht ein besseres Netzvermögen als die meisten Verbindungen aus dem Stand der Technik aufweisen.

Die erfindungsgemäßen Verbindungen der allgemeinen Formel (II) sind jedoch auch - was mit der vorliegenden Erfindung hauptsächlich angestrebt war - hervorragend biologisch abbaubar. Sogar im GF-Test (GF = geschlossene Flasche), der im Vergleich zu den sonst üblichen Prüfmethoden sehr viel höhere Anforderungen an die biologische Abbaubarkeit der getesteten Verbindungen stellt, werden sehr gute Ergebnisse erreicht: Die Verbindungen weisen $BSB_{30}$-Werte von z.T. deutlich über 60 % auf. Die Verbindungen gemäß der Erfindung erfüllen damit die geltenden gesetzlichen Vorschriften. Eine Beeinträchtigung der Abwässer bei Verwendung der erfindungsgemäßen Verbindungen der allgemeinen Formel (II) ist also nicht zu befürchten.

Von der vorliegenden Erfindung werden auch Mischungen mehrerer Verbindungen der allgemeinen Formel (II) er faßt; die gemäß dem nachfolgend beschriebenen Herstellungsverfahren erhalten werden. Mischungen mehrerer Verbindungen (II) entstehen insbesondere dann, wenn schon Vorstufen im Rahmen des industriellen Verfahrens in Form von Mischungen mehrerer gleichartiger Verbindungen anfallen: die Weiterbehandlung solcher Mischungen führt dann nicht zu einem einheitlichen Reaktionsprodukt, sondern wiederum zu Produktmischungen, deren Bestandteile jedoch homologe Strukturen aufweisen und such in ihren Eigenschaften nur geringfügig unterscheiden. So werden beispielsweise Mischungen von Verbindungen (II) von der vorliegenden Erfindung mit umfaßt, die einen unterschiedlichen Ethoxylierungsgrad aufweisen, in denen also n in der allgemeinen Formel (II) über einen mehr oder weniger großen Bereich streut.

Die sulfatierten Hydroxyalkylpolyethylenglykolether der allgemeinen Formel (II) sowie auch ihre Mischungen werden dadurch hergestellt, daß man Epoxide der allgemeinen Formel (III)

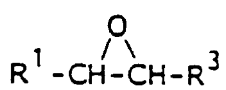

(III)

in der $R^1$ und $R^3$ die oben angegebenen Bedeutungen haben, mit Alkoholalkoxylaten der allgemeinen Formel (IV)

$R^2\text{-}O(CH_2CH_2)_nH$ (IV)

in der $R^2$ und n die oben angegebenen Bedeutungen haben, bei erhöhter Temperatur in Gegenwart eines Katalysators umsetzt, die Verbindungen (I) oder deren Mischungen gegebenenfalls in an sich bekannter Weise aus dem Reaktionsgemisch isoliert, die so erhaltenen Verbindungen (I) oder deren Mischungen bei erhöhter Temperatur mit einem Sulfatierungsreagenz zur Umsetzung bringt, das rohe Sulfierprodukt in eine wäßrige basische Lösung einträgt und das Gemisch bei erhöhter Temperatur hält, das Gemisch auf einen pH-Wert im neutralen bzw. schwach alkalischen Bereich bringt und die so erhaltenen Produkte (II) oder deren Mischungen gewünschtenfalls in an sich bekannter Weise aus dem Reaktionsgemisch isoliert.

Ausgangsstoffe des Verfahrens zur Herstellung der Verbindungen der allgemeinen Formel (II) sind also einerseits Epoxide der allgemeinen Formel (III). Derartige Epoxide entstehen aus auf petrochemischem Wege in großen Mengen erhältlichen Olefinen, in der Mehrzahl der Fälle Monoolefinen, durch an sich bekannte Epoxidationsreaktionen, beispielsweise durch Umsetzungen der genannten Olefine mit Percarbonsäuren oder ähnlichen, Epoxide bildenden Reagenzien. Epoxide (III) im Sinne der vorliegenden Erfindung können 1,2-Epoxide sein. In derartigen endständigen Epoxiden, die erfindungsgemäß bevorzugt sind, steht $R^3$ für Wasserstoff, und $R^1$ in der allgemeinen Formel (III) steht für einen linearen Alkylrest mit 6 bis 16, bevorzugt mit 8 bis 12 C-Atomen. Es ist jedoch in dem erfindungsgemäßen Verfahren auch möglich, Epoxide mit nicht endständigen Oxiranringen in die Reaktion einzusetzen.

Reaktionspartner der oben beschriebenen Epoxide der allgemeinen Formel (III) sind Alkoholethoxylate der allgemeinen Formel (IV). Derartige Verbindungen (IV) entstehen auf an sich bekanntem Wege aus

Alkoholen und Olefinepoxiden. Im vorliegenden Fall werden unter Alkoholethoxylaten der allgemeinen Formel (IV) die Verbindungen verstanden, die aus den entsprechenden Alkoholen und Ethylenoxid entstehen. Bevorzugte Reaktionspartner in dem vorliegenden Verfahren sind Alkoholethoxylate der allgemeinen Formel (IV), in der $R^2$ für lineare, gesättigte Alkylreste mit 1 bis 22 C-Atomen steht. Die für diese Bedeutung von $R^2$ möglichen Alkylreste sind oben im Zusammenhang mit den Verbindungen der allgemeinen Formel (II) aufgeführt. Mit Vorteil sind die Verbindungen verwendbar, in denen $R^2$ in der allgemeinen Formel (IV) einen gesättigten linearen Alkylrest mit 1 bis 12 C-Atomen, bevorzugt mit 1 bis 4 C-Atomen bedeutet. Das heißt im Rahmen der vorliegenden Erfindung, daß zur Herstellung der Edukte für die Herstellung der erfindungsgemäß einzusetzenden Alkoholethoxylate der allgemeinen Formel (IV) am meisten bevorzugt Alkohole mit 1 bis 4 C-Atomen mit Alkylenoxiden wie Ethylenoxid umgesetzt werden. Darunter fallen also in diesem Fall Alkohole aus der Gruppe Methanol, Ethanol, n-Propanol und n-Butanol. Alkohole aus dieser Gruppe werden - in diesem bevorzugten Fall - mit Ethylenoxid umgesetzt, wobei dann die besonders bevorzugten Alkoholethoxylate der allgemeinen Formel (IV) entstehen. Das Umsetzungsverhältnis, das auch letztlich die Zahl der Ethoxygruppen im Molekül der Verbindungen der allgemeinen Formel (IV) bestimmt, liegt im Bereich 1 : 1 bis 1 : 30, so daß n im Zahlenmittel einen Wert im Bereich von 1 bis 30 annimmt. Ein bevorzugter Bereich für n ist der von 1 bis 12.

Entsprechend dem erfindungsgemäßen Verfahren zur Herstellung der Verbindungen der allgemeinen Formel (II) wird bei der Umsetzung von (III) mit (IV) ein Molverhältnis im Bereich von ca. 1 : 1 eingestellt.

Die genannte Umsetzung wird bei erhöhter Temperatur durchgeführt. Dies bedeutet erfindungsgemäß, daß das Reaktionsgemisch - unter Umständen unter Schutzgas -auf Reaktionstemperaturen im Bereich von 100 bis 180 ° C erhitzt wird, bei denen dann die Umsetzung in hinreichender Geschwindigkeit mit befriedigender Ausbeute stattfindet. Dabei wird ein Temperaturbereich von 120 bis 160 ° C bevorzugt.

Das Verfahren zur Herstellung der Hydroxyalkylpolyethylenglykole der allgemeinen Formel (I) wird in Gegenwart eines Katalysators durchgeführt. Ausreichend zur Durchführung der Reaktion ist eine Katalysatormenge im Bereich von 0.01 bis 2.0 Gew.-%, bezogen auf das Gesamtgewicht des Reaktionsgemisches. Es können erfindungsgemäß sowohl saure als auch basische Katalysatoren verwendet werden. Bevorzugte Katalysatorsysteme sind Alkalimetallalkoholate, Mineralsäuren wie $H_2SO_4$ oder Lewis-Säuren wie $BF_3$-Etherat. Bevorzugter Katalysator in dem erfindungsgemäßen Verfahren ist Natriummethylat.

Der erste Schritt des Verfahrens gemäß der Erfindung wird durch das nachfolgende Reaktionsschema verdeutlicht.

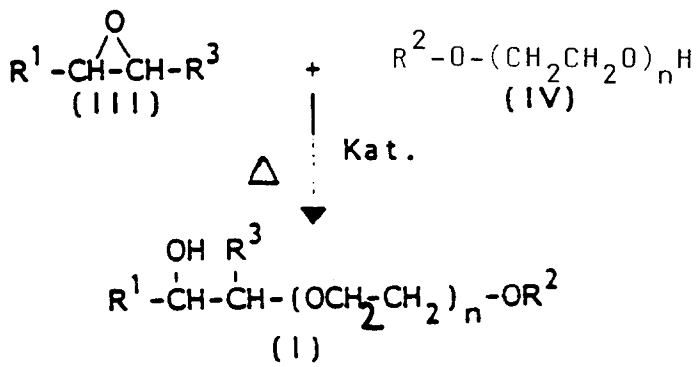


Wie voranstehend schon angedeutet wurde, entstehen im ersten Schritt des erfindungsgemäßen Verfahrens entsprechend dem vorstehenden Reaktionsschema nicht nur Einzelverbindungen der allgemeinen Formel (I), sondern auch Mischungen derartiger Verbindungen. Dies resultiert zum einen daraus, daß in bestimmten Fällen die Öffnung des Oxiranrings des eingesetzten Epoxids in zwei verschiedenen Richtungen erfolgen kann und da durch die Bildung unterschiedlicher Produkte möglich ist, zum anderen jedoch auch daraus, daß schon als Edukte unter Umständen Substanzgemische eingesetzt werden. So führt die Umsetzung der Alkohole mit Ethylenoxid in bestimmten Molverhältnissen nicht zu einem einheitlichen Produkt: vielmehr entstehen Produktgemische verschiedener Verbindungen (IV) mit einer mehr oder weniger breiten Verteilung der Zahl der Alkoxygruppen im Molekül, d.h. einer mehr oder weniger breiten Streuung von n in der allgemeinen Formel (IV). Der Einsatz von Gemischen der Alkoholethoxylate (IV) in das erfindungsgemäße Verfahren muß dann auch ein Gemisch von Produkten (I) zur Folge haben Auch solche Produktgemische sowie Verfahren zur Herstellung derartiger Gemische werden von der vorliegenden Erfindung mit umfaßt.

Der zweite Schritt des erfindungsgemäßen Verfahrens besteht in der Umsetzung der in der ersten Verfahrensstufe erhaltenen Verbindungen (I) oder deren Mischungen mit einem Sulfatierungsreagenz. Diese Reaktion findet bei erhöhter Temperatur statt. So wird die Sulfatierungsreaktion - je nach dem verwendeten Sulfatierungsreagenz - mit Vorteil im Temperaturbereich von 10 bis 40 ° C durchgeführt.

Als Sulfatierungsreagenzien können in dem erfindungsgemäßen Verfahren eine Vielzahl von Verbindungen eingesetzt werden. Mit besonderem Vorteil werden als Sulfatierungsreagenzien Chlorsulfonsäure oder - was besonders bevorzugt ist - gasförmiges Schwefeltrioxid verwendet. Gasförmiges $SO_3$ wird beispielsweise dadurch in das Reaktionssystem, das die Verbindung (I) oder deren Mischungen enthält, eingeführt, daß man es, bei spielsweise mit Hilfe eines Tauchrohres, aus Oleum, also überkonzentrierter bzw. mit $SO_3$ angereicherter Schwefelsäure, austreibt.

Bei der Durchführung des erfindungsgemäßen Verfahrens hat es sich als zweckmäßig erwiesen, die Verbindungen der allgemeinen Formel (I) mit dem Sulfatierungsreagenz in einem Molverhältnis von 1 : 1 bis 1 : 1,3 und vorzugsweise in einem Molverhältnis von 1 : 1,05 bis 1 : 1,1 umzusetzen.

In einer bevorzugten Ausführungsform des erfindungsgemäßen Verfahrens läßt sich der Reaktionsverlauf dadurch steuern, daß man das als Sulfatierungsreagenz verwendete gasförmige Schwefeltrioxid mit unter den Reaktionsbedingungen inerten Gasen verdünnt. Dadurch wird insbesondere eine Temperatursteuerung der Reaktion erleichtert.

Als unter Reaktionsbedingungen inerte Gase lassen sich im Rahmen des erfindungsgemäßen Verfahrens vorteilhaft Luft oder Stickstoff verwenden. Es werden mit besonderem Vorteil Gasmischungen von $SO_3$ mit Luft oder Stickstoff verwendet, in denen der Anteil von $SO_3$ im Bereich von 1 bis 10 Vol.-% liegt. Damit läßt sich ohne Schwierigkeiten eine Temperaturführung im Bereich von 10 bis 40 ° C erreichen.

Der oben beschriebene zweite Reaktionsschritt des erfindungsgemäßen Verfahrens wird durch das nachfolgende Reaktionsschema verdeutlicht:

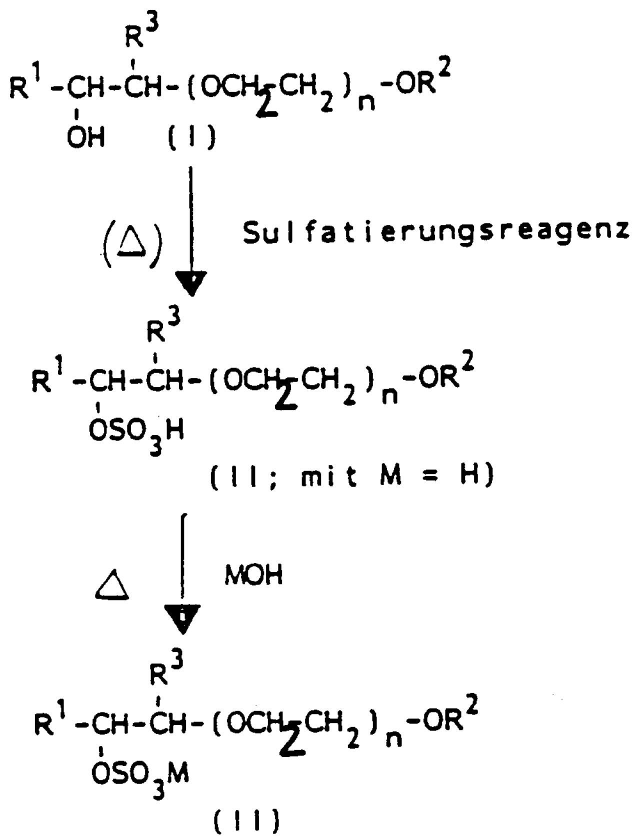


Entsprechend dem erfindungsgemäßen Verfahren wird nachfolgend das rohe Sulfierprodukt in eine wäßrige, basische Lösung eingetragen und das Gemisch anschließend bei erhöhter Temperatur gehalten. In einer bevorzugten Ausführungsform wird als wäßrige, basische Lösung eine Alkalimetallhydroxide, Ammoniak, ein oder mehrere Alkylamine mit 1 bis 4 C-Atomen pro Alkylgruppe oder ein oder mehrere Alkanolamine mit 1 bis 4 C-Atomen pro Alkanolrest enthaltende Lösung vorgelegt, in die das rohe Sulfierprodukt sukzessive eingetragen wird. Mit besonderem Vorteil wird wäßrige Natronlauge oder Kalilauge oder eine

Lösung mit Ammoniak, einem oder mehreren $C_1$ - und/oder $C_2$-Alkylaminen und/oder einem oder mehreren $C_1$ und/oder $C_2$-Alkanolaminen als basischen Komponenten verwendet. Bei Verwendung am moniakalischer oder Amine enthaltender basischer Lösungen tritt anstelle eines Metallatoms in der allgemeinen Formel (II) die entsprechende Ammoniumgruppe. Bei der erfindungsgemäß bevorzugten Verwendung wäßriger, Alkalimetallhydroxide, insbesondere NaOH oder KOH enthaltender Lösungen wird die Konzentration an Alkalimetallhydroxid so gewählt, daß sie im Bereich von 1,0 bis 1,3 Mol Alkalimetallhydroxid pro Mol angelagertes $SO_3$ liegt. Diese Konzentration in Alkalimetallhydroxid in der vorgelegten Lösung hat den Vorteil, daß nicht nur eine vollständige Überführung des sauren Sulfatierungsproduktes in das entsprechende Alkalimetallsalz stattfindet, sondern auch die im nachfolgenden Verfahrensschritt erfolgende pH-Weit-Einstellung nur eine begrenzte Säurezugabe erfordert.

Nach dem Eintrag des rohen Sulfierproduktes in die wäßrige basische Lösung wird das Reaktionsgemisch eine Zeit lang bei erhöhter Temperatur gehalten. Bevorzugt liegt diese Temperatur im Bereich von 60 bis 100 ° C, wobei sich in der Praxis ein Wert von ungefähr 95 ° C über eine Zeit von ca. 30 min bewährt hat.

Der voranstehend beschriebene Reaktionsschritt wird durch das obige Reaktionsschema näher verdeutlicht.

Vor der weiteren Verwendung der auf diesem Wege erhaltenen Verfahrensprodukte der allgemeinen Formel (II) wird das Reaktionsgemisch auf einen pH-Wert im neutralen oder schwach alkalischen Bereich gebracht.

Dies geschieht im einfachsten Falle dadurch, daß man das im voranstehend beschriebenen Verfahrensschritt erhaltene Reaktionsgemisch mit verdünnten Mineralsäuren, beispielsweise mit verdünnter Schwefelsäure, neutralisiert. Anschließend kann, sofern dies gewünscht wird, das so erhaltene Produkt (II) oder eine Mischung mehrerer derartiger Verbindungen aus dem Reaktionsgemisch isoliert werden.

Gegenüber Verfahren aus dem Stand der Technik weist das vorliegende Verfahren, abgesehen von der Bildung neuer und gegenüber dem Stand der Technik verbesserter Produkte (II), den wesentlichen Vorteil auf, daß aus einfach in guten Ausbeuten zugänglichen Edukten die Verbindungen der allgemeinen Formel (II) auch im industriellen Maßstab hergestellt werden können. Dabei werden gute Sulfiergrade erreicht, und es entstehen die gewünschten Verbindungen in hoher Qualität und Reinheit, wenn man von der Bildung von Produktmischungen absieht, die die Verwendbarkeit der erhaltenen Verbindungen (II) in keiner Weise einschränken.

Die wie oben beschrieben herstellbaren Verbindungen der allgemeinen Formel (II) oder auch die verfahrensgemäß erhaltenen Mischungen derartiger Verbindungen werden als Netzmittel und Waschmittelrohstoffe verwendet. Für diese Verwendung weisen die erhaltenen Verbindungen (II) den Vorteil auf, daß sie schaumarm sind.

Darüber hinaus lassen sich die nach dem erfindungsgemäßen Verfahren hergestellten Verbindungen der allgemeinen Formel (II) vollständig biologisch abbauen. Aufgrund der linearen Endgruppen sind sie einem mikrobiellen Abbau schon in relativ kurzer Zeit zugänglich und lassen eine langfristige Belastung des Wassers nicht befürchten. Derartige Verbindungen enthaltende Abwässer belasten die Umwelt somit nicht.

Die sulfatierten Hydroxy-Mischether der Formel II weisen auch eine befriedigende Haut- und Schleimhautverträglichkeit auf. Sie eignen sich daher auch als Waschrohstoffe für kosmetische Wasch- und Reinigungsmittel. Mit anderen, schaumstarken, in der Kosmetik üblichen Tensiden, z.B. mit Fettalkoholsulfaten und Fettalkoholpolyglykolethersulfaten weisen sie eine gute Verträglichkeit auf. Solche Gemische aus erfindungsgemäßen sulfatierten Hydroxy-Mischethern und Fettalkoholsulfaten oder Fettalkoholpolyglykolethersulfaten auf Basis von linearen Fettalkoholen mit 12 bis 18 C-Atomen oder Anlagerungsprodukten von 1 bis 12 Mol Ethylenoxid an solche Fettalkohole eignen sich z.B. als Waschrohstoffe für die Herstellung von Flüssigseifen, Shampoos, Dusch- und Badepräparaten.

Insbesondere wurde festgestellt, daß wäßrige Lösungen von Mischungen aus erfindungsgemäßen sulfatierten Hydroxyalkylpolyethylenglykolethern der Formel II und Fettalkoholsulfaten und/oder Fettalkoholpolyglykolethersulfaten ein besonders gutes Schaumverhalten aufweisen, da der Schaum auch gegen Kalkseifenbelastung wesentlich stabiler ist, als der Schaum, den die Komponenten an sich gegenüber Kalkseifenbelastung zeigen. Dies ist von besonderem Vorteil in Zusammensetzungen, die Seifen enthalten oder die gemeinsam mit Seife in hartem Wasser angewendet werden, ohne daß der Schaum dadurch beeinträchtigt werden soll. Ein Beispiel für eine solche Anwendung sind Schaumbadezusätze, deren Schaum in hartem Wasser - auch nach Benutzung von Fettsäure-Seife - weitgehend erhalten bleiben soll.

Die Fettalkoholsulfate und Fettalkoholpolyglycolethersulfate, die diese synergistische Wirkung in Mischungen mit den erfindungsgemäßen sulfatierten Hydroxyalkylpolyethylenglykolethern der Formel II zeigen sind bevorzugt solche der Formel V

$R^5$-(O-$CH_2$-$CH_2$)$_x$-$OSO_3$A (V)

in welcher $R^5$ eine Alkylgruppe mit 10 bis 16 C-Atomen ist, X = 0 oder eine Zahl von 1 bis 10 und A ein Ammoniumion, Mono-, Di und Trialkanolammoniumion mit 2 bis 4 C-Atomen in der Alkanolgruppe, eine Alkali-oder ein Magnesiumion sein kann. Die Alkylgruppe $R^5$ ist bevorzugt eine primäre, lineare Alkylgruppe mit 12 bis 14 C-Atomen. Die Mischungen können im Gewichtsverhältnis Hydroxy-Mischether-Sulfat (II) : Alkylethersulfat (V) = 10 : 90 bis 90 : 10, bevorzugt im Gewichtsverhältnis 10 : 90 bis 50 : 50 eingesetzt werden.

Die Erfindung wird durch die nachfolgenden Beispiele näher erläutert. Im Beispielteil werden folgende Abkürzungen verwendet:
SG = Sulfiergrad;
TR = Trockenrückstand.
AS = Aktivsubstanz (Trockenrückstand abzüglich anorganischer Salze und unsulfierter Anteile);
US = unsulfonierter Anteil:
WAS = Waschaktivsubstanz (Sulfonat- und Sulfatanteil);
Alle Prozentangaben sind in Gew.-%, sofern nicht ausdrücklich anders angegeben.

Beispiel 1

Als Ausgangsmaterial diente ein Umsetzungsprodukt aus 1 Mol 1,2-Epoxyoctan und 1 Mol Anlagerungsprodukt von 10 Mol Ethylenoxid an n-Butanol.

In einem Labor-Standreaktor, der mit 162,6 g 0.282 Mol) des Ausgangsmaterials gefüllt und auf 35 ° C erwärmt war, wurden innerhalb von 15 min 23,7 g (0,296 Mol) gasförmiges (aus 36,4 g Oleum ausgetriebenes) Schwefeltrioxid über ein Tauchrohr eingeleitet. Das Reaktionsgemisch wurde mit 24,8 g (0,311 Mol) einer 50 gew.-%igen wäßrigen Lösung von Natriumhydroxid neutralisiert und anschließend 30 min bei 95 ° C gerührt. Zuletzt wurde mit verdünnter Schwefelsäure auf einen pH-Wert von 7,5 eingestellt und der verwendete Indikator (Phenolphthalein) mit 1 ml einer 13 gew.-%igen wäßrigen Natriumhypochloritlösung zerstört.

Folgende Analysendaten wurden ermittelt:

| | |
|---|---|
| SG | 85,6 % |
| TR | 33,4 % |
| AS | 31,1 % |
| WAS | 26,6 % |
| US | 3,8 % |
| NaCl | 0,07 % |
| $Na_2SO_4$ | 2,2 % |

Beispiel 2

Als Ausgangsmaterial wurde ein Umsetzungsprodukt aus 1 Mol 1,2-Epoxytetradecan und 1 Mol Anlagerungsprodukt von 10 Mol Ethylenoxid an n-Butanol eingesetzt.

In einem Planschliffkolben wurden 286,5 g (0,5 Mol) des Ausgangsmaterials vorgelegt. Durch eine Tropftrichter mit Druckausgleich und verlängerter Tropfspitze (Tauchrohr) wurden innerhalb von 20 min 61,2 g (0,525 Mol) Chlorsulfonsäure durch einen schwachen Argonstrom eingetragen, wobei die Temperatur 30 ° C nicht überschritt. Das Reaktionsgemisch wurde mit 44,1 g einer 50 gew.-%igen wäßrigen Lösung von Natriumhydroxid neutralisiert und auf 750 g Paste aufgefüllt. Folgende Anlaysendaten wurden ermittelt:

| | |
|---|---|
| SG | 96,1 % |
| TR | 48,9 % |
| AS | 47,6 % |
| WAS | 46,2 % |
| $Na_2SO_4$ | 1,3 % |

Beispiele 3 bis 68

Entsprechend der in den Beispielen 1 und 2 beschriebenen Vorgehensweise wurden weitere Verbindungen der allgemeinen Formel (II) hergestellt, die in der nachfolgenden Tabelle 1 charakterisiert sind.

TABELLE 1

Beispiele 3-62: Hergestellte Verbindungen der Formel II ($R^3$ = H)

| Beispiel | $R^1$ | $R^2$ | n | M | SC % | TR % | AS % | US % | WAS % |
|---|---|---|---|---|---|---|---|---|---|
| 3 | $C_{10}$ | $C_1$ | 1 | Na | 95,5 | 46,2 | 42,4 | 1,1 | 37,2 |
| 4 | $C_{12}$ | $C_1$ | 1 | Na | 87,3 | 36,5 | 31,8 | 3,0 | 27,8 |
| 5 | $C_6$ | $C_1$ | 2 | Na | 97,1 | 64,3 | 61,6 | 1,1 | 52,5 |
| 6 | $C_8$ | $C_1$ | 2 | Na | 96,7 | 53,7 | 51,5 | 1,0 | 42,5 |
| 7 | $C_{10}$ | $C_1$ | 2 | Na | 97,1 | 43,9 | 42,2 | 0,8 | 36,3 |
| 8 | $C_{12}$ | $C_1$ | 2 | Na | 93,3 | 36,7 | 34,9 | 1,6 | 29,1 |
| 9 | $C_{14}$ | $C_1$ | 2 | Na | 85,0 | 51,3 | 47,5 | 5,6 | 40,4 |
| 10 | $C_{16}$ | $C_1$ | 2 | Na | 89,5 | 43,2 | 39,8 | 3,5 | 36,7 |
| 11 | $C_6$ | $C_1$ | 4 | Na | 97,3 | 68,1 | 66,6 | 1,4 | 60,2 |
| 12 | $C_8$ | $C_1$ | 4 | Na | 96,3 | 65,0 | 62,4 | 1,5 | 50,6 |
| 13 | $C_{14}$ | $C_1$ | 4 | Na | 86,0 | 58,2 | 56,1 | 6,4 | 47,8 |
| 14 | $C_{16}$ | $C_1$ | 4 | Na | 77,8 | 68,2 | 65,5 | 13,9 | 58,6 |
| 15 | $C_{10}$ | $C_1$ | 5 | Na | 98,1 | 46,7 | 45,0 | 0,6 | 38,1 |
| 16 | $C_{12}$ | $C_1$ | 5 | Na | 96,0 | 53,7 | 52,0 | 1,5 | 44,0 |

TABELLE 1 (Fortsetzung)

| Beispiel | $R^1$ | $R^2$ | n | M | SC % | TR % | AS % | US % | WAS % |
|---|---|---|---|---|---|---|---|---|---|
| 17 | $C_6$ | $C_1$ | 10 | Na | 95,7 | 50,3 | 49,2 | 1,6 | 42,1 |
| 18 | $C_{10}$ | $C_1$ | 10 | Na | 95,8 | 53,9 | 53,0 | 1,7 | 45,8 |
| 19 | $C_{14}$ | $C_1$ | 10 | Na | 94,8 | 48,2 | 47;0 | 2,1 | 44,2 |
| 20 | $C_8$ | $C_1$ | 11 | Na | 86,3 | 77,0 | 71,1 | 8,7 | 63,9 |
| 21 | $C_{12}$ | $C_1$ | 11 | Na | 89,8 | 77,6 | 75,0 | 6,6 | 66,4 |
| 22 | $C_{16}$ | $C_1$ | 11 | Na | 88,9 | 51,2 | 49,1 | 5,4 | 45,1 |
| 23 | $C_6$ | $C_4$ | 1 | Na | 96,9 | 48,4 | 45,7 | 0,9 | 40,7 |
| 24 | $C_8$ | $C_4$ | 1 | Na | 97,3 | 51,0 | 48,4 | 0,9 | 44,6 |
| 25 | $C_{10}$ | $C_4$ | 1 | Na | 88,8 | 41,5 | 39,5 | 3,4 | 35,8 |
| 26 | $C_{12}$ | $C_4$ | 1 | Na | 83,7 | 33,0 | 31,4 | 4,1 | 27,5 |
| 27 | $C_6$ | $C_4$ | 2 | Na | 98,7 | 57,7 | 54,3 | 0,5 | 51,3 |
| 28 | $C_8$ | $C_4$ | 2 | Na | 97,5 | 52,3 | 49,3 | 0,9 | 47,1 |
| 29 | $C_{10}$ | $C_4$ | 2 | Na | 95,8 | 55,1 | 51,9 | 1,6 | 48,2 |
| 30 | $C_{12}$ | $C_4$ | 2 | Na | 86,9 | 51,3 | 48,1 | 5,2 | 44,3 |
| 31 | $C_{12}$ | $C_4$ | 2 | K | 98,7 | 52,7 | 51,3 | 0,5 | 51,7 |

TABELLE 1 (Fortsetzung)

| Beispiel | $R^1$ | $R^2$ | n | M | SG % | TR % | AS % | US % | WAS % |
|---|---|---|---|---|---|---|---|---|---|
| 32 | $C_{12}$ | $C_4$ | 2 | Li | 97,3 | 50,7 | 48,1 | 1,1 | 50,3 |
| 33 | $C_{12}$ | $C_4$ | 2 | DEA* | 98,4 | 77,4 | 60,1 | 0,6 | 58,4 |
| 34 | $C_{14}$ | $C_4$ | 2 | Na | 85,3 | 36,9 | 34,5 | 4,4 | 32,2 |
| 35 | $C_{16}$ | $C_4$ | 2 | Na | 80,9 | 31,8 | 29,7 | 5,1 | 26,5 |
| 36 | $C_6$ | $C_4$ | 4 | Na | 96,9 | 62,1 | 60,5 | 1,5 | 60,9 |
| 37 | $C_{14}$ | $C_4$ | 4 | Na | 91,5 | 39,1 | 38,1 | 2,8 | 37,2 |
| 38 | $C_6$ | $C_4$ | 5 | Na | 96,0 | 51,3 | 48,9 | 1,5 | 45,5 |
| 39 | $C_8$ | $C_4$ | 5 | Na | 95,2 | 53,3 | 51,2 | 1,9 | 47,1 |
| 40 | $C_{10}$ | $C_4$ | 5 | Na | 81,3 | 55,5 | 53,7 | 8,4 | 44,6 |
| 41 | $C_{12}$ | $C_4$ | 5 | Na | 84,1 | 57,0 | 55,3 | 7,6 | 48,3 |
| 42 | $C_{14}$ | $C_4$ | 5 | Na | 85,2 | 27,0 | 25,9 | 3,2 | 22,1 |
| 43 | $C_{16}$ | $C_4$ | 5 | Na | 89,1 | 48,4 | 46,7 | 4,3 | 42,2 |
| 44 | $C_8$ | $C_4$ | 10 | Na | 83,5 | 39,7 | 38,2 | 5,3 | 31,4 |
| 45 | $C_{10}$ | $C_4$ | 10 | Na | 78,8 | 38,9 | 37,9 | 7,1 | 30,4 |
| 46 | $C_{12}$ | $C_4$ | 10 | Na | 80,8 | 44,7 | 43,7 | 7,5 | 36,6 |
| 47 | $C_{14}$ | $C_4$ | 10 | Na | 79,6 | 49,1 | 47,8 | 8,8 | 39,4 |

T A B E L L E 1 (Fortsetzung)

| Beispiel | $R^1$ | $R^2$ | n | M | SG % | TR % | AS % | US % | WAS % |
|---|---|---|---|---|---|---|---|---|---|
| 48 | $C_{16}$ | $C_4$ | 10 | Na | 76,1 | 45,9 | 44,4 | 9,9 | 35,8 |
| 49 | $C_{10}$ | $C_4$ | 15 | Na | 95,5 | 48,1 | 47,2 | 1,9 | 45,2 |
| 50 | $C_{14}$ | $C_4$ | 19 | Na | 91,7 | 55,4 | 54,8 | 4,6 | 54,4 |
| 51 | $C_6$ | $C_4$ | 27 | Na | 91,9 | 45,7 | 45,1 | 3,7 | 45,1 |
| 52 | $C_{14}$ | $C_4$ | 27 | Na | 88,2 | 52,8 | 52,2 | 6,6 | 52,6 |
| 53 | $C_6$ | $C_8$ | 2 | Na | 97,0 | 54,6 | 52,5 | 1,3 | 54,9 |
| 54 | $C_{10}$ | $C_8$ | 4 | K | 97,5 | 45,4 | 44,5 | 0,9 | 44,4 |
| 55 | $C_{10}$ | $C_8$ | 4 | Li | 97,9 | 40,2 | 39,4 | 0,7 | 38,4 |
| 56 | $C_{10}$ | $C_8$ | 4 | DEA* | 98,0 | 61,8 | 59,3 | 0,8 | 52,2 |
| 57 | $C_{14}$ | $C_8$ | 9 | Na | 94,9 | 50,7 | 49,6 | 2,1 | 44,4 |
| 58 | $C_{12}$ | $C_{12}/C_{13}$ | 2 | Na | 81,6 | 32,5 | 31,3 | 5,4 | 29,1 |
| 59 | $C_{10}$ | $C_{12}/C_{13}$ | 7 | Na | 87,5 | 50,3 | 48,8 | 5,6 | 45,7 |
| 60 | $C_{12}$ | $C_{12}/C_{13}$ | 7 | Na | 87,7 | 36,2 | 35,1 | 3,9 | 32,3 |
| 61 | $C_6$ | $C_{12}/C_{14}$ | 4 | Na | 92,3 | 66,0 | 64,0 | 4,3 | 62,7 |

TABELLE 1 (Fortsetzung)

| Beispiel | $R^1$ | $R^2$ | n | M | SC % | TR % | AS % | US % | WAS % |
|---|---|---|---|---|---|---|---|---|---|
| 62 | $C_{10}$ | $C_{16}/C_{18}$ | 11 | Na | 92,5 | 47,1 | 46,3 | 3,2 | 45,0 |

*) Diethanolammonium

Netzvermögen

Das Netzvermögen der entsprechend den Beispielen 1 bis 4 erhaltenen Verbindungen wurde entsprechend der Vorschrift nach DIN 53 901 bestimmt. Die Ergebnisse sind der nachfolgenden Tabelle 2 zu

entnehmen.

Tabelle 2

| Netzvermögen nach DIN 53 901 | |
|---|---|
| Verbindung aus Beispiel Nr. | Netzzeit (sec) |
| 1 | >300 |
| 2 | 96 |
| 39 | 58 |
| 40 | 17 |
| 41 | 24 |
| 42 | 55 |
| 44 | 100 |
| 45 | 61 |
| 46 | 87 |
| 47 | 130 |
| 48 | 198 |
| 23 | 51 |
| 24 | 38 |
| 25 | 16 |
| 26 | 41 |
| 27 | 66 |
| 28 | 50 |
| 29 | 12 |
| 30 | 23 |
| 34 | 56 |
| 38 | 191 |
| 43 | 132 |
| 3 | 134 |
| 4 | 49 |
| 7 | 166 |
| 8 | 127 |
| 59 | 174 |
| 60 | >300 |
| 58 | >300 |

Schaumvermögen

In einem gesonderten Test wurde das Schaumvermögen der erfindungsgemäßen Verbindungen der allgemeinen Formel (II) bestimmt. Dazu wurden jeweils 100 ml einer wäßrigen Lösung der Verbindungen der allgemeinen Formel (II) (Konzentration: 1 Gew.-% AS) in einem 250 ml-Standzylinder mit Graduierung fünfmal geschüttelt. Das Schaumvolumen wurde jeweils sofort nach dem Schüttelvorgang, nach 1, 3 und 5 min abgelesen.

Es wurde für die Herstellung der Tensidlösungen Wasser mit 0 ° dH und mit 16 ° dH verwendet.

Die Ergebnisse sind der nachfolgenden Tabelle 3 zu entnehmen.

Tabelle 3

| Verb. aus Bsp. Nr. | Schaummenge (ml) | | | | | | | |
|---|---|---|---|---|---|---|---|---|
| | sofort | | nach 1' | | nach 3' | | nach 5' | |
| | 0 ° dH | 16 ° dH | 0 ° dH | 16 ° dH | 0 ° dH | 16 ° dH | 0 ° dH | 16 ° dH |
| 1 | 230 | 230 | 180 | 180 | 170 | 170 | 150 | 160 |
| 2 | 210 | 200 | 70 | 120 | 10 | 50 | 8 | 20 |
| 39 | 300 | 300 | 250 | 250 | 250 | 220 | 180 | 180 |
| 40 | 250 | 250 | 180 | 200 | 140 | 170 | 75 | 150 |
| 41 | 250 | 200 | 150 | 140 | 105 | 110 | 40 | 60 |
| 42 | 100 | 120 | 60 | 70 | 30 | 30 | 20 | 20 |
| 44 | 200 | 180 | 145 | 125 | 130 | 120 | 120 | 120 |
| 45 | 200 | 200 | 150 | 150 | 130 | 140 | 70 | 110 |
| 46 | 200 | 200 | 100 | 110 | 10 | 80 | 10 | 40 |
| 47 | 110 | 120 | 10 | 50 | 6 | 25 | 2 | 10 |
| 48 | 90 | 100 | 25 | 45 | 20 | 40 | 20 | 30 |
| 23 | 300 | 300 | 180 | 200 | 140 | 170 | 70 | 160 |
| 24 | 300 | 300 | 250 | 250 | 180 | 200 | 150 | 180 |
| 25 | 300 | 300 | 210 | 210 | 200 | 190 | 180 | 170 |
| 26 | 180 | 160 | 140 | 120 | 120 | 90 | 90 | 70 |
| 27 | 300 | 300 | 250 | 250 | 230 | 230 | 150 | 170 |
| 28 | 300 | 300 | 180 | 250 | 150 | 200 | 50 | 180 |
| 29 | 300 | 300 | 210 | 200 | 210 | 150 | 210 | 100 |
| 30 | 180 | 180 | 130 | 130 | 70 | 100 | 40 | 70 |
| 34 | 120 | 120 | 70 | 70 | 50 | 50 | 50 | 50 |
| 38 | 300 | 250 | 250 | 200 | 200 | 180 | 170 | 150 |
| 43 | 80 | 100 | 30 | 40 | 30 | 30 | 30 | 30 |
| 3 | 300 | 300 | 250 | 250 | 200 | 200 | 190 | 190 |
| 4 | 300 | 300 | 200 | 190 | 160 | 160 | 110 | 100 |
| 7 | 220 | 200 | 170 | 150 | 150 | 130 | 70 | 90 |
| 8 | 140 | 140 | 80 | 70 | 45 | 25 | 25 | 20 |
| 59 | 140 | 160 | 90 | 110 | 70 | 110 | 50 | 80 |
| 60 | 140 | 140 | 40 | 70 | 30 | 70 | 30 | 50 |
| 58 | 120 | 120 | 60 | 60 | 30 | 50 | 30 | 40 |

Biologische Abbaubarkeit

Fünf der gemäß den Beispielen 1 bis 68 hergestellten Verbindungen wurden hinsichtlich ihrer biologischen Abbaubarkeit im sogenannten GF-Test (GF = geschlossene Flasche) untersucht. Dieser Test ist beschrieben in W.K. Fischer. "Zeitschrift Tenside/Detergents" 8, 182 (1971).

Die Ergebnisse sind der nachfolgenden Tabelle 4 zu entnehmen.

Tabelle 4

| Testergebnisse des GF-Tests auf biologische Abbaubarkeit | |
|---|---|
| Verb. aus Bsp. | $BSB_{30}$/COD (%) |
| 38 | 55 |
| 43 | > 60 |
| 60 | > 60 |
| 58 | > 60 |

Ergebnis:

Entsprechend der Bewertung des literaturbeschriebenen GF-Tests sind also von den fünf aufgeführten Verbindungen

- die des Beispiels 38 als gut und
- die der restlichen drei Beispiele als sehr gut biologisch abbaubar einzustufen.

Prüfung der Kalkseifenbelastbarkeit des Schaumes

Es wurden Schaummessungen mit wäßrigen Lösungen (Konzentration - 2 g/l) bei 20 ° C gemäß DIN 53902 (Schaumschlag-Prüfung) durchgeführt. Die Messungen wurden bei 0° dH, 11° dH, 22° dH und unter Kalkseifenbelastung bei 22° dH in Gegenwart von 0,5 g/l Natriumoleat durchgeführt.

Die Schlagschaum-Bestimmungen wurden für die erfindungsgemäßen Hydroxy-Mischether-Sulfate der Beispiele 3, 7 und 15, für ein Alkylethersulfat (AES) und für Mischungen dieser Komponenten im Gewichtsverhältnis 30 : 70 durchgeführt.

Als Alkylethersulfat (AES) wurde ein technisches Alkylethersulfat-Natriumsalz verwendet, daß durch Sulfatierung eines Anlagerungsproduktes von 2 Mol Ethylenoxyd an ein lineares Lauryl-Myristylalkohol-Gemisch (70 : 30) hergestellt war (Formel V : $R^5 = C_{12}H_{25}/C_{14}H_{29}$, x = 2, A = Na).

Die Ergebnisse sind der folgenden Tabelle 5 zu entnehmen.

**Tabelle 5**

| Tenside | Schaummengen (ml) | | | |
|---|---|---|---|---|
| | 0° dH | 11° dH | 22° dH | 22° dH/Na-Oleat |
| Substanz aus Beispiel 3 | 670 | 590 | 510 | 170 |
| Substanz aus Beispiel 7 | 700 | 580 | 540 | 70 |
| Substanz aus Beispiel 15 | 630 | 550 | 520 | 90 |
| Alkylethersulfat (AES) | 830 | 840 | 850 | 180 |
| 30% Beispiel 3 + 70% AES | | | | 420 |
| 30% Beispiel 7 + 70% AES | | | | 370 |
| 30% Beispiel 15 + 70% AES | | | | 300 |

## Patentansprüche

**1.** Sulfatierte Hydroxyalkylpolyethylenglycolether der allgemeinen Formel (II)

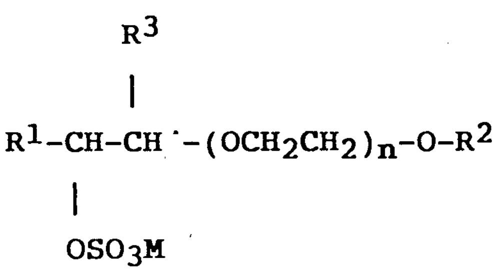

(II)

in der

- $R^1$ für einen linearen Alkylrest mit 6 bis 16 C-Atomen,
- $R^2$ für einen linearen oder verzweigten, gesättigten Alkylrest mit 1 bis 22 C-Atomen,
- $R^3$ für Wasserstoff oder einen linearen Alkylrest mit 1 bis 16 C-Atomen,
- M für Wasserstoff, Ammonium, Alkylammonium, Alkanolammonium, worin die Alkyl- und Alkanolreste je 1 bis 4 C-Atome haben, oder ein einwertiges Metallatom und

n für Zahlen von 1 bis 30
steht, mit der Maßgabe, daß die Gesamtzahl der in $R^1$ und $R^3$ enthaltenen C-Atome 6 bis 16 beträgt.

**2.** Verfahren zur Herstellung von sulfatierten Hydroxyalkylpolyethylenglycolethern der allgemeinen Formel (II)

$$\begin{array}{l} \qquad R^3 \\ \qquad | \\ R^1\text{-}CH\text{-}CH\text{-}(OCH_2CH_2)_n\text{-}O\text{-}R^2 \\ \quad | \\ \quad OSO_3M \end{array} \qquad (II)$$

in der
- $R^1$ für einen linearen Alkylrest mit 6 bis 16 C-Atomen,
- $R^2$ für einen linearen oder verzweigten, gesättigten Alkylrest mit 1 bis 22 C-Atomen,
- $R^3$ für Wasserstoff oder einen linearen Alkylrest mit 1 bis 16 C-Atomen,
- M für Wasserstoff, Ammonium, Alkylammonium, Alkanolammonium, worin die Alkyl- und Alkanolreste je 1 bis 4 C-Atome haben, oder ein einwertiges Metallatom und
- n für Zahlen von 1 bis 30

steht, mit der Maßgabe, daß die Gesamtzahl der in $R^1$ und $R^3$ enthaltenen C-Atome 6 bis 16 beträgt, bei dem man
a) Epoxide der allgemeinen Formel **(III)**,

$$\begin{array}{l} \qquad O \\ \quad / \ \backslash \\ R^1\text{-}CH\text{-}CH\text{-}R^3 \end{array} \qquad (III)$$

in der $R^1$ und $R^3$ die oben angegebenen Bedeutungen haben, mit Alkoholethoxylaten der allgemeinen Formel **(IV)**,

$$R^2\text{-}O\text{-}(CH_2CH_2O)_nH \qquad (IV)$$

in der $R^2$ und n die oben angegebenen Bedeutungen haben, bei erhöhter Temperatur in Gegenwart eines Katalysators umsetzt,
b) das Reaktionsprodukt bei erhöhter Temperatur mit einem Sulfatierungsreagenz behandelt ,
c) das Sulfatierungsprodukt in eine wäßrige basische Lösung einträgt, das Gemisch bei erhöhter Temperatur hält und anschließend auf einen pH-Wert im neutralen oder schwach alkalischen Bereich einstellt.

**3.** Verfahren nach Anspruch 2, **dadurch gekennzeichnet**, daß man die Epoxide und Alkoholethoxylate im molaren Verhältnis von ca. 1 : 1 einsetzt.

**4.** Verfahren nach Anspruch 2, **dadurch gekennzeichnet**, daß man die Reaktion bei Temperaturen von 100 bis 180 °C durchführt.

**5.** Verfahren nach Anspruch 2, **dadurch gekennzeichnet**, daß man den Katalysator in einer Menge von 0,01 bis 2,0 Gew.-% - bezogen auf das Gesamtgewicht des Reaktionsgemisches - einsetzt.

**6.** Verfahren nach Anspruch 2, **dadurch gekennzeichnet**, daß man als Katalysatoren Alkalimetallalkoholate, Schwefelsäure oder Bortrifluoridetherat einsetzt.

7. Verfahren nach Anspruch 2, **dadurch gekennzeichnet**, daß man als Sulfatierungsreagenzien gasförmiges Schwefeltrioxid oder Chlorsulfonsäure einsetzt.

8. Verfahren nach Anspruch 2, **dadurch gekennzeichnet**, daß man die Sulfatierungsreaktion bei Temperaturen von 10 bis 40 ° C durchführt.

9. Verfahren nach Anspruch 2, **dadurch gekennzeichnet**, daß man das rohe Sulfatierungsprodukt nach dem Eintragen in die wäßrigen basischen Lösungen bei einer Temperatur von 60 bis 100 ° C hält.

10. Verwendung von sulfatierten Hydroxyalkylpolyethylenglycolethern nach Anspruch 1 als Netzmittel sowie Rohstoffe für Wasch- und Reinigungsmittel.

**Claims**

1. Sulfated hydroxyalkyl polyethylene glycol ethers corresponding to general formula (II):

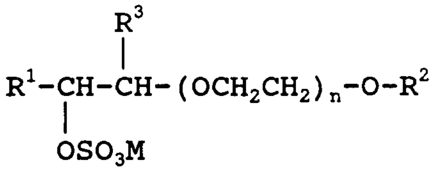

(I)

in which

- $R^1$ is a linear alkyl radical containing 6 to 16 carbon atoms,
- $R^2$ is a linear or branched, saturated alkyl radical containing 1 to 22 carbon atoms,
- $R^3$ is hydrogen or a linear alkyl radical containing 1 to 16 carbon atoms,
- M is hydrogen, ammonium, alkylammonium, alkanolammonium, in which the alkyl and alkanol groups each contain 1 to 4 carbon atoms, or a monovalent metal atom and
- n is a number of 1 to 30,

with the proviso that the total number of carbon atoms in $R^1$ and $R^3$ is 6 to 16.

2. A process for the production of sulfated hydroxyalkyl polyethylene glycol ethers corresponding to general formula (II):

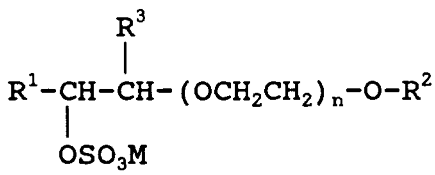

(I)

in which

- $R^1$ is a linear alkyl radical containing 6 to 16 carbon atoms,
- $R^2$ is a linear or branched, saturated alkyl radical containing 1 to 22 carbon atoms,
- $R^3$ is hydrogen or a linear alkyl radical containing 1 to 16 carbon atoms,
- M is hydrogen, ammonium, alkylammonium, alkanolammonium, in which the alkyl and alkanol groups each contain 1 to 4 carbon atoms, or a monovalent metal atom and
- n is a number of 1 to 30,

with the proviso that the total number of carbon atoms in $R^1$ and $R^3$ is 6 to 16,
in which

a) epoxides corresponding to general formula (III):

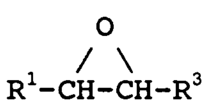

(III)

in which $R^1$ and $R^3$ are as defined above,
are reacted with alcohol ethoxylates corresponding to general formula (IV):

$R^2$-O-($CH_2CH_2O)_nH$ (IV)

in which $R^2$ and n are as defined above, at elevated temperature in the presence of a catalyst,
b) the reaction product is treated with a sulfating agent at elevated temperature,
c) the sulfation product is introduced into an aqueous basic solution, the mixture is kept at elevated temperature and is then adjusted to a pH value in the neutral or mildly alkaline range.

**3.** A process as claimed in claim 2, characterized in that the epoxides and alcohol ethoxylates are used in a molar ratio of approximately 1:1.

**4.** A process as claimed in claim 2, characterized in that the reaction is carried out at temperatures of 100 to 180 °C.

**5.** A process as claimed in claim 2, characterized in that the catalyst is used in a quantity of 0.01 to 2.0% by weight, based on the total weight of the reaction mixture.

**6.** A process as claimed in claim 2, characterized in that alkali metal alcoholates, sulfuric acid or boron trifluoride etherate are used as catalysts.

**7.** A process as claimed in claim 2, characterized in that gaseous sulfur trioxide or chlorosulfonic acid is used as the sulfating agent.

**8.** A process as claimed in claim 2, characterized in that the sulfation reaction is carried out at temperatures of 10 to 40 °C.

**9.** A process as claimed in claim 2, characterized in that the crude sulfation product is kept at a temperature of 60 to 100 °C after introduction into the aqueous basic solutions.

**10.** The use of the sulfated hydroxyalkyl polyethylene glycol ethers claimed in claim 1 as wetting agents and as raw materials for detergents and cleaning products.

## Revendications

**1.** Ethers sulfates d'hydroxyalkylpolyéthylèneglycol de la formule générale (II)

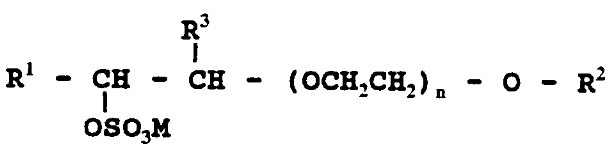 (II)

dans laquelle
$R^1$ représente un radical alkyle linéaire avec 6 à 16 atomes de carbone,
$R^2$ représente un radical alkyle linéaire ou ramifié, saturé avec 1 à 22 atomes de carbone,
$R^3$ représente un atome d'hydrogène ou un radical alkyle linéaire avec 1 à 16 atomes de carbone,
M représente un atome d'hydrogène, un radical ammonium, alkylammonium, alcanolammonium, dans lequel le radical alkyle et le radical alcanol possède chacun 1 à 4 atomes de carbone, ou un atome d'un métal monovalent, et
n représente un nombre de 1 à 30 étant entendu que le nombre total des atomes de carbone contenus dans $R^1$ et $R^3$ est de 6 à 16.

**2.** Procédé de préparation des éthers sulfates d'hydroxyalkylpolyéthylèneglycol de la formule générale (II)

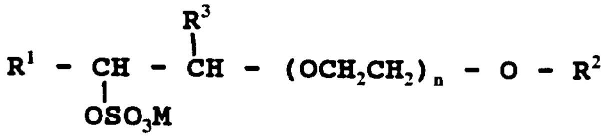 (II)

dans laquelle

$R^1$ représente un radical alkyle linéaire avec 6 à 16 atomes de carbone,

$R^2$ représente un radical alkyle linéaire ou ramifié, saturé avec 1 à 22 atomes de carbone,

$R^3$ représente un atome d'hydrogène ou un radical alkyle linéaire avec 1 à 16 atomes de carbone,

M représente un atome d'hydrogène, un radical ammonium, alkylammonium, alcanolammonium, dans lequel le radical alkyle et le radical alcanol possède chacun 1 à 4 atomes de carbone, ou un atome d'un métal monovalent, et

n représente un nombre de 1 à 30 étant entendu que le nombre total des atomes de carbone contenus dans $R^1$ et $R^3$ est de 6 à 16, par lequel on transforme

a) un époxyde de la formule générale (III),

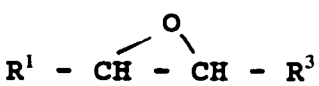 (III)

dans laquelle $R^1$ et $R^3$ ont les significations ci-dessus mentionnées, avec des éthoxylates d'alcool de la formule générale (IV),

**$R^2 - O - (CH_2CH_2O)_nH$ (IV)**

dans laquelle $R^2$ et n ont les significations ci-dessus mentionnées, à des températures élevées en présence d'un catalyseur,

b) le produit de la réaction est traité à haute température avec un réactif de transformation en sulfate,

c) le sulfate est introduit dans une solution aqueuse basique, le mélange est maintenu à température élevée et ensuite amené à une valeur de pH dans une zone neutre ou faiblement alcaline.

**3.** Procédé selon la revendication 2, caractérisé en ce que l'époxyde et l'éthoxylate d'alcool sont utilisés dans une proportion molaire d'environ 1:1.

**4.** Procédé selon la revendication 2, caractérisé en ce que la réaction est effectuée à des températures de 100 à 180 °C.

**5.** Procédé selon la revendication 2, caractérisé en ce qu'on utilise le catalyseur dans une quantité de 0,01 à 2,0% en poids - par rapport au poids total du mélange de la réaction.

**6.** Procédé selon la revendication 2, caractérisé en ce qu'on utilise comme catalyseur un alcoolate des métaux alcalins, l'acide sulfurique ou un éthérate de borotrifluorure.

**7.** Procédé selon la revendication 2, caractérisé en ce qu'on utilise comme réactif de transformation en sulfate du trioxyde de soufre gazéiforme ou de l'acide chlorosulfonique.

**8.** Procédé selon la revendication 2, caractérisé en ce que la réaction de transformation en sulfate est effectuée à des températures de 10 à 40 °C.

**9.** Procédé selon la revendication 2, caractérisé en ce qu'on maintient le sulfate brut, après son introduction dans la solution aqueuse basique, à une température de 60 à 100 °C.

**10.** Utilisation des éthers sulfates d'hydroxyalkylpolyéthylèneglycol selon la revendication 1 comme agent mouillant aussi bien que comme produit brut dans les produits de lessive ou de nettoyage.